# NEW EUROPEAN PATENT SPECIFICATION

(11) **EP 1 225 930 B2**
(45) Date of publication and mention of the opposition decision: **26.10.2016**
(45) Mention of the grant of the patent: 22.11.2006
(21) Application number: 00962566.6
(22) Date of filing: 29.09.2000
(51) Int. Cl.: A61M 1/00

(54) **SUCTION BAG ASSEMBLY**
SAUGBEUTELANORDNUNG
ENSEMBLE MUNI D'UN SAC DE PRELEVEMENT PAR ASPIRATION

(30) Priority: 01.10.1999 FI 992123
(43) Date of publication of application: 31.07.2002
(73) Proprietor: Serres Oy, 61850 Kauhajoki as. (FI)
(72) Inventor: RAJAMÄKI, Veikko, FIN-61800 Kauhajoki (FI)
(74) Representative: Kolster Oy Ab
(86) International application number: PCT/FI2000/000840
(87) International publication number: WO 2001/024846

(56) References cited:
- WO-A1-94/14045
- US-A- 4 228 798
- US-A- 4 460 361
- US-A- 4 681 571

## Description

The invention relates to a suction bag assembly according to the preamble of claim 1 and a suction bag structure according to the preamble of claim 9 adapted insertable into a collection container of said suction bag assembly.

In the surgical, intensive-care and first-aid departments of hospitals, for instance, the collection of different fluids sucked from a patient has conventionally been carried out using collection containers, wherein the fluid is sucked by a vacuum. Generally, the vacuum source is connected to the collection container via a tubing so that the interior of the collection container can be brought to a vacuum. Also another tubing, commonly known as the patient tubing, is passed from the container to the point selected for sucking fluid. When the vacuum source is operative, the fluid being sucked moves along the patient tubing under the vacuum into the collection container. When full, the container is emptied, washed and put in use again.

Due to hygienic reasons, washable collection containers have been replaced by disposable suction bags, generally made from a polymer material, that can be air-tightly sealed with the help of a cover into the interior of a collection container. The connector of the patient tubing is adapted to the cover of the container, while the connector of the tubing communicating with the vacuum source may be adapted to either the cover or the side of the container. Not only the interior of the suction bag, but also the space remaining between the interior surface of the collection container and the exterior surface of the suction bag must be brought to a vacuum that expands the bag against the walls of the collection container and thus prevents the flexible suction bag from collapsing under the vacuum brought into its interior.

Suction bag systems commonly used today can be categorized by their construction into two major types. In the first type, the suction bag has thereto connected in a permanent manner a thick and solid cover portion that also serves as the cover of the collection container after the suction bag has been inserted into the container. The bag and the cover portion may be attached to each other by welding, for instance. When the bag is full, the bag with its integral cover is removed from the container so that a new bag with its integral cover can be inserted therein. This kind of collection bag structure is disclosed in US Pat. No. 4,516,973, for instance.

In the other major type, the basic structure of the system is formed by a separate cover cooperating with a collection container, while the suction bag is attached to the cover portion by quick-connects, for instance. When the suction bag is full, the bag is detached from the cover, whereupon a new bag can be attached thereto. This kind of suction bag structure is disclosed in application publication EP 861,668, for instance.

Generally in both of the above-described suction bag systems, there is located between the collection container and the vacuum source a filter that serves to prevent microbes from entering the vacuum system. Additionally, the system is commonly provided with a suction bag overfill preventer that may be implemented integrally with the filter by way of inserting therein material which undergoes swelling when wetted. In this manner, the filter can be arranged to block the conduit to the suction system when the fluid level in the suction bag reaches the filter level, thus preventing the entry of fluid into the suction system. Conventionally, the filter is located to operate in a suction opening of the suction bag or in conjunction with a vacuum connector mounted on the cover portion.

In order to ensure correct and safe operation of a suction bag system, it is important to prevent inadvertent misconnection of the patient/vacuum tubings to wrong connectors. The risk of such a misconnection is high, particularly in systems wherein the suction bag and the cover portion are permanently connected to each other. If the connectors for both of the tubings are located in the cover portion, the tubings must be disconnected and reconnected each time the suction bag is replaced, as, for example, in the solution disclosed in US Pat. No. 4,460,361. Obviously, the risk of misconnection of tubings can be prevented by way of using connectors of different types or dimensions for the patient tubing and the vacuum system tubing. As the connector types of suction bag systems used in different countries may be unlike each other, a wide selection of incompatible connector systems can be found in commercial fluid collection equipment.

To ease the replacement of a suction bag, suction bag collection systems have been developed based on suction bag structures comprising an integral combination of a cover portion with a suction bag, whereby the replacement of the full bag only needs the patient tubing to be disconnected and then reconnected to the cover portion of the new bag. Herein, the vacuum system tubing is attached to a connector adapted to the side of the collection container, thus dispensing with the need to disconnect the vacuum tubing at the replacement of the bag. In a known construction of this kind, the vacuum is brought into the suction bag via an opening provided at the side of the bag. A disadvantage of such a structure, however, is that the combination filter-overfill preventer protecting the vacuum system must be mounted to the flexible and thin bag portion, which is very awkward in series production. The structure of this kind of suction bag is described in US Pat. No. 4,516,973, for instance.

In publication WO 94/14045 is described a construction, comprising the features of the preamble of claims 1 and 9, respectively, wherein the opening of the collection container is sealed with a detachable cover portion. The suction bag is attached in a permanent manner to another cover portion. The cover portions of the collection container and the suction bag are connected to each other with the help of support spacers so that a gap remains therebetween. The cover portion of the suction bag has an opening that is covered by a filter from the side facing the cover portion of the collection container of the suction bag. The filter is held in place by means of a lattice that can be inserted between the two cover portions. The patient tubing is attached to a connector extending through the cover portions and the vacuum system tubing is attached to the side of the collection container so that the vacuum is applied via the interior of the opposed cover portions into the interior of the suction bag. A disadvantage of this construction is that the cover construction is complicated and expensive to manufacture inasmuch it comprises three separate components, namely, the cover of the collection container, the cover of the suction bag and the lattice supporting the filter therebetween when the filter is inserted between the opposed cover portions. After the filter is properly fixed, the cover portions must be joined to each other, which adds to the worksteps and production costs encountered in manufacture.

In collection systems, wherein the suction bag is connected to the cover portion by means of, e.g., quick-connects, an advantage is that the tubings attached to the connectors of the cover portion need not necessarily be disconnected during the replacement of the suction bag inasmuch the same cover portion detached from a full suction bag can be directly reused with a new bag. However, as the seal in the cover of this type of construction wears with time, the sealed joint between the cover portion and the collection container begins to leak, whereby the vacuum system becomes ineffective.

It is an object of the invention to provide an entirely novel type of suction bag assembly capable of overcoming the disadvantages of the above-described techniques.

In the suction bag assembly according to the invention, the connector for the vacuum system tubing is adapted to the side of the collection container, while the connector for the patient tubing is adapted to the cover portion that also has the suction bag attached thereto. This arrangement needs only the patient tubing connected to the cover to be disconnected when the suction bag is being replaced. The vacuum is brought via the vacuum system connector to the space remaining between the exterior surface of the suction bag and the interior surface of the collection container wall and also to the interior of the suction bag via a conduit adapted to the cover portion. The filter protecting the vacuum system, also serving as an overfill protector, is adapted into a conduit made in the cover portion, particularly to that end of the conduit which exits into the interior of the suction bag.

More specifically, the suction bag assembly according to the invention is characterized by what is stated in the characterizing part of claim 1.

Furthermore, the suction bag structure according to the invention is characterized by what is stated in the characterizing part of claim 9.

The invention offers significant benefits.

The suction bag assembly according to the invention is free from the risk of misconnection of the patient tubing and the vacuum system tubing to wrong connectors inasmuch only the connector of the patient tubing must be disconnected during the replacement of the suction bag. Moreover, the construction according to the invention dispenses with the need for differently dimensioned connectors to prevent inadvertent mistakes in the connection of tubings. Furthermore, the replacement of a full suction bag is a quick operation. In the suction bag assembly according to the invention, the filter protecting the vacuum system is adapted to the underside interior surface of the cover portion which is a significantly superior location as compared to the conventional placement of the filter at the side of the bag or between two joined cover parts.

In the following, the invention is examined in detail by making reference to the attached drawing wherein
FIG. 1 is a longitudinally sectional view of a suction bag assembly according to the invention; and

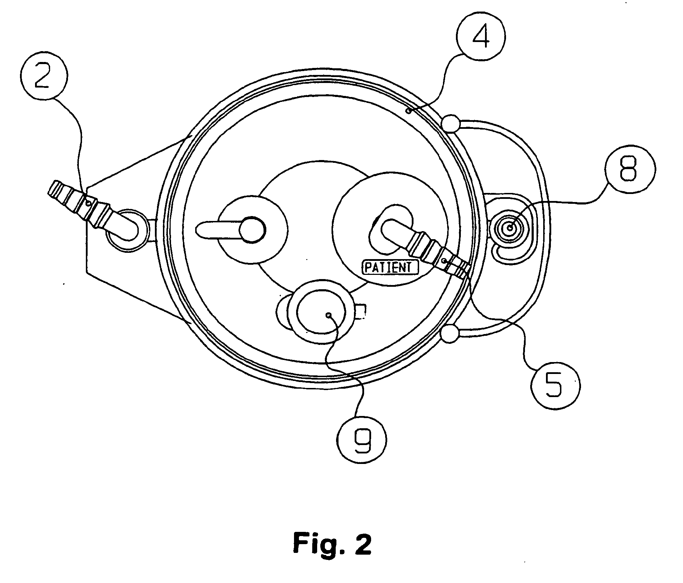

FIG. 2 is a top view of the suction bag assembly of FIG. 1.

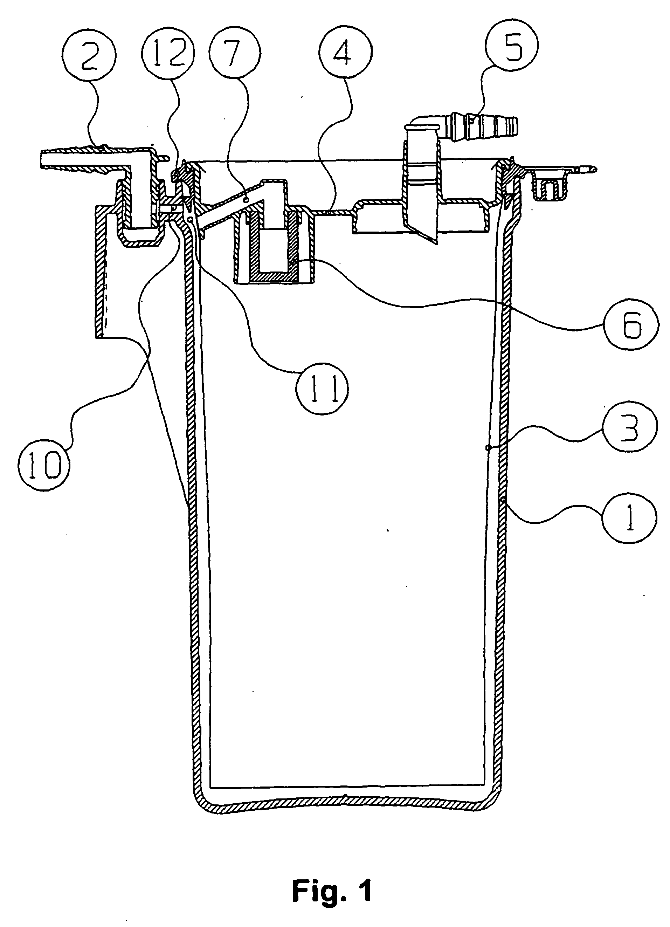

Referring to FIG. 1, the suction bag assembly shown therein comprises a collection container 1, made from a durable polymer material, having adapted to its support handle a vacuum connector 2, whereto a tubing passed to a vacuum source can be connected. The vacuum connector 2 has a conduit 10 exiting on the interior surface of the collection container 1.

To the collection container 1 is placed a suction bag 3 made from a material that is impervious to fluid and air. The suction bag 3 is advantageously attached permanently to a cover 4 by welding, for instance. The cover 4 is stiffer and thicker than the suction bag 3 and can be made from a polymer material, for instance. The cover has a connector 5, whereto a tubing can be connected passed to a patient or other point, wherefrom fluid is to be sucked. Additionally, the cover 4 has attached thereto with a flexible tie a plug 8, by means of which the patient tubing connector 5 can be plugged when the suction bag 3 is full. Additionally, there is adapted to the cover 4 a sampling/drainage connector 9 for the sucked fluid so as to make the connector channel to extend through the cover 4. The connector 9 may also be used for feeding therethrough a disinfectant and/or a solidifying agent into the suction bag 3 or for a series connection of multiple suction bags. The cover 4 is shaped to have a rim 12 adapted to be tightly compressible against the rim of the collection container 1 so as to form a seal that maintains the vacuum applied between the interior surface of the collection container 1 and the exterior surface of the suction bag 3 and, respectively, in the space 11 between the interior surface of the cover 4 and the interior surface of the collection container 1. The seal in this joint may be implemented, e.g., using O-rings mountable about the rim of the cover 4.

The cover 4 has a conduit 7 whose first end exits on the exterior surface of the cover 4, into the space 11 remaining between the interior surface of the collection container 1 and the cover 4, while its second end exits into the interior of the suction bag 3 facing the cover 4. On the cover 4, at the second end of the conduit 7, there is adapted a filter 6 serving to prevent contaminants from entering the vacuum system. Additionally, the filter 6 is advantageously made self-sealing under a contact with a fluid, thus preventing the entry of fluid into the conduit 7 when the fluid level in the suction bag 3 reaches the level of the filter 6.

In the use of the suction bag system, a vacuum is applied via a tubing communicating with a vacuum source via the conduit 10 of the vacuum connector 2 into the space between the interior surface of the collection container 1 and the exterior surface of the suction bag 3 and, respectively, into the space 11 between the cover 4 and the interior surface of the collection container 1, whereby the vacuum is further applied via the conduit 7 of the cover 4 and the filter 6 thereof into the suction bag 3 and therefrom further to the patient tubing connector 5 and, finally, into the suction tubing connected thereto, thus forcing the fluid to flow from the point subjected to sucking toward the suction bag 3. Accordingly, the flow of the vacuum in the conduit 7 takes place from the suction bag 3 toward the vacuum connector 2. When the fluid level in the suction bag 3 reaches the level of the filter 6, the filter 6 cuts off the fluid flow, thus preventing the entry of fluid into the vacuum system. After the suction bag 3 is sucked full, both the suction bag 3 with the cover 4 is removed from the collection container 1 so that a new suction bag 3 with its integral cover 4 can be inserted in the collection container 1.

As the conduit 7 and the filter 6 are dimensioned to cause some pressure loss in the vacuum flow, the vacuum prevailing in the space between the interior surface of the collection container 1 and the exterior surface of the suction bag 3 exceeds the vacuum prevailing in the interior of the suction bag, thus preventing the suction bag 3 from collapsing, particularly when empty.

## Claims

1. Suction bag assembly for collection of liquid fluid, the assembly comprising
- a collection container (1) open at its one end,
- a cover (4) for closing said one open end of said collection container (1) and adapted to the inserted into said collection container (1), a flexible suction bag (3) attached to said cover (4),
- a vacuum connector (2) provided at said collection container (1) and communicating via a flow channel with a vacuum source so as to provide a vacuum between the interior surface of said collection container (1) and the exterior surface of said suction bag (3),
- a patient tubing connector (5) provided at said cover (4) and serving to provide fluid communications from the source of fluids to the interior of said suction bag (3),
- a conduit (7) provided at said cover (4) for transmitting the applied vacuum from the space (11) remaining between the interior surface of said collection container (1) and the cover (4) to the interior of said suction bag (3), and
- a filter (6) serving to prevent contaminants from entering the vacuum system,
**characterized in that**
- said filter (6) is adapted to a surface of the cover (4) facing the interior of the suction bag (3),
- said filter is adapted to the end of said conduit exiting into the interior of said suction bag,
- said cover (4) is a unitary part.

2. Assembly according to claim 1, **characterized in that** the direction of flow of vacuum in the conduit (7) takes place from the suction bag (3) toward the vacuum connector (2).

3. Assembly according to claim 1, **characterized in that** said suction bag (3) is permanently attached to said cover (4).

4. Assembly according to claim 1, **characterized in that** said filter (6) is made of a material capable of swelling when wetted.

5. Assembly according to claim 1, **characterized in that** said vacuum connector (2) is adapted to the support handle of said collection container (1).

6. Assembly according to claim 1, **characterized by** a plug (8) attached to said cover (4) with a flexible tie so as to serve as a plug for said patient tubing connector (5).

7. Assembly according to claim 1, **characterized by** a sampling/drainage connector (9) adapted to said cover (4) for handling the sucked fluid.

8. Assembly according to claim 1, **characterized in that** said vacuum connector (2) incorporates a conduit (10) via which the vacuum can be brought into the interior of said collection container (1).

9. Suction bag structure insertable into a collection container (1) of liquid fluids which is open at its one end and incorporates a vacuum connector (2) suited for communicating with a vacuum source, the suction bag structure comprising
- a cover (4) for closing said one open end of said collection container (1),
- a flexible suction bag (3) attached to said cover (4),
- a patient tubing connector (5) provided at said cover and serving to provide fluid communications from the source of fluids to the interior of said suction bag (3),
- a conduit (7) provided at said cover (4) for transmitting the applied vacuum from the space (11) remaining between the interior surface of said collection container (1) and the cover (4) to the interior of said suction bag (3), and
- a filter (6) serving to prevent contaminants from entering the vacuum system,
**characterized in that**
- said filter (6) is adapted to a surface of the cover (4) facing the interior of the suction bag (3),
- said filter is adapted to the end of said conduit exiting into the interior of said suction bag,
- said cover (4) is a unitary part.

## Patentansprüche

1. Saugbeutelanordnung zur Sammlung eines flüssigen Fluids, wobei die Anordnung umfaßt
- einen Sammelbehälter (1), der an seinem einen Ende offen ist,
- eine Abdeckung (4) zum Verschließen des genannten einen offenen Endes des genannten Sammelbehälters (1) und angepaßt, um in den genannten Sammelbehälter (1) eingesetzt zu werden, wobei ein flexibler Saugbeutel (3) an der genannten Abdeckung (4) befestigt ist,
- einen Unterdruckanschluß (2), der an dem genannten Sammelbehälter (1) vorgesehen ist und über einen Strömungskanal mit einer Unterdruckquelle in Verbindung steht, um einen Unterdruck zwischen der inneren Oberfläche des genannten Sammelbehälters (1) und der äußeren Oberfläche des genannten Saugbeutels (3) bereitzustellen,
- einen Patientenschlauchanschluß (5), der an der genannten Abdeckung (4) vorgesehen ist und dazu dient, eine Strömungsverbindung von der Quelle von Fluiden zu der Innenseite des genannten Saugbeutels (3) bereitzustellen,
- eine Leitung (7), die an der genannten Abdeckung (4) vorgesehen ist, um den aufgebrachten Unterdruck von dem Zwischenraum (11), der zwischen der inneren Oberfläche des genannten Sammelbehälters (1) und der Abdeckung (4) verbleibt, zu dem Inneren des genannten Saugbeutels (3) zu übertragen, und
- einen Filter (6), der dazu dient, Verunreinigungen daran zu hindern, in das Unterdrucksystem einzutreten,
**dadurch gekennzeichnet, daß**
- der Filter (6) an eine Oberfläche der Abdeckung (4), die zu dem Inneren des Saugbeutels (3) weist, angepaßt ist,
- der genannte Filter an das Ende der genannten Leitung, die in das Innere des genannten Saugbeutels abführt, angepaßt ist,
- die genannte Abdeckung (4) ein einteiliges Element ist.

2. Anordnung nach Anspruch 1, **dadurch gekennzeichnet, daß** die Strömungsrichtung des Unterdrucks in der Leitung (7) ausgehend von dem Saugbeutel (3) in Richtung auf den Unterdruckanschluß (2) erfolgt.

3. Anordnung nach Anspruch 1, **dadurch gekennzeichnet, daß** der genannte Saugbeutel (3) ständig an der genannten Abdeckung (4) befestigt ist.

4. Anordnung nach Anspruch 1, **dadurch gekennzeichnet, daß** der genannte Filter (6) aus einem Material hergestellt ist, das in der Lage ist, anzuschwellen, wenn es befeuchtet wird.

5. Anordnung nach Anspruch 1, **dadurch gekennzeichnet, daß** der genannte Unterdruckanschluß (2) an den Traggriff des genannten Sammelbehälters (1) angepaßt ist.

6. Anordnung nach Anspruch 1, **dadurch gekennzeichnet, daß** ein Stopfen (8) mit einem flexiblen Band an der genannten Abdeckung (4) befestigt ist, so daß er als ein Stopfen für den genannten Patientenschlauchanschluß (5) dient.

7. Anordnung nach Anspruch 1, **dadurch gekennzeichnet, daß** ein Probenahme- bzw. Abzugsanschluß (9) an die genannte Abdeckung (4) angepaßt ist, um das abgesaugte Fluid zu behandeln.

8. Anordnung nach Anspruch 1, **dadurch gekennzeichnet, daß** der genannte Unterdruckanschluß (2) eine Leitung (10) enthält, über die der Unterdruck in das Innere des genannten Sammelbehälters (1) gebracht werden kann.

9. Saugbeutelstruktur, die in einen Sammelbehälter (1) für flüssige Fluide eingesetzt werden kann, der an seinem einen Ende offen ist und einen Unterdruckanschluß (2) aufweist, der geeignet ist, um mit einer Unterdruckquelle in Verbindung zu treten, wobei die Saugbeutelstruktur umfaßt
- eine Abdeckung (4), um das genannte eine offene Ende des genannten Sammelbehälters (1) zu verschließen,
- einen flexiblen Saugbeutel (3), der an der genannten Abdeckung (4) befestigt ist,
- einen Patientenschlauchanschluß (5), der an der genannten Abdeckung (4) vorgesehen ist und dazu dient, eine Strömungsverbindung von der Quelle von Fluiden zu der Innenseite des genannten Saugbeutels (3) bereitzustellen,
- eine Leitung (7), die an der genannten Abdeckung (4) vorgesehen ist, um den aufgebrachten Unterdruck von dem Zwischenraum (11), der zwischen der inneren Oberfläche des genannten Sammelbehälters (1) und der Abdeckung (4) verbleibt, zu dem Inneren des genannten Saugbeutels (3) zu übertragen, und
- einen Filter (6), der dazu dient, Verunreinigungen daran zu hindern, in das Unterdrucksystem einzutreten,
**dadurch gekennzeichnet, daß**
- der Filter (6) an eine Oberfläche der Abdeckung (4), die zu dem Inneren des Saugbeutels (3) weist, angepaßt ist,
- der genannte Filter an das Ende der genannten Leitung, die in das Innere des genannten Saugbeutels abführt, angepaßt ist,
- die genannte Abdeckung (4) ein einteiliges Element ist.

## Revendications

1. Ensemble muni d'un sac de prélèvement par aspiration pour recueillir un fluide liquide, l'ensemble comprenant:
- un récipient collecteur (1) ouvert à l'une de ses extrémités,
- un couvercle (4) pour fermer ladite une extrémité ouverte dudit récipient collecteur (1) et adapté pour être inséré dans ledit récipient collecteur (1),
- un sac souple (3) de prélèvement par aspiration fixé audit couvercle (4),
- un raccord de vide (2) prévu au niveau dudit récipient collecteur (1) et communiquant via un canal d'écoulement avec une source de vide afin de fournir un vide entre la surface intérieure dudit récipient collecteur (1) et la surface extérieure dudit sac (3) de prélèvement par aspiration,
- un raccord (5) pour tubulure patient prévu au niveau dudit couvercle (4) et servant à fournir des communications fluidiques à partir de la source de fluides jusqu'à l'intérieur dudit sac (3) de prélèvement par aspiration,
- un conduit (7) prévu au niveau dudit couvercle (4) pour transmettre le vide appliqué depuis l'espace (11) restant entre la surface intérieure dudit récipient collecteur (1) et le couvercle (4) jusqu'à l'intérieur dudit sac (3) de prélèvement par aspiration, et
- un filtre (6) servant à prévenir toute pénétration de contaminants dans le système de vide, **caractérisé en ce que**
- ledit filtre (6) est adapté à une surface du couvercle (4) tournée vers l'intérieur du sac (3) de prélèvement par aspiration,
- ledit filtre est adapté à l'extrémité dudit conduit débouchant dans l'intérieur dudit sac de prélèvement par aspiration,
- ledit couvercle (4) est une pièce unitaire.

2. Ensemble selon la revendication 1, **caractérisé en ce que** le sens d'écoulement du vide dans le conduit (7) s'effectue du sac (3) de prélèvement par aspiration vers le raccord de vide (2).

3. Ensemble selon la revendication 1, **caractérisé en ce que** ledit sac (3) de prélèvement par aspiration est fixé de façon permanente audit couvercle (4).

4. Ensemble selon la revendication 1, **caractérisé en ce que** ledit filtre (6) est fabriqué à partir d'un matériau capable de gonfler lorsqu'il est mouillé.

5. Ensemble selon la revendication 1, **caractérisé en ce que** ledit raccord de vide (2) est adapté à la poignée de support dudit récipient collecteur (1).

6. Ensemble selon la revendication 1, **caractérisé par** un bouchon (8) fixé audit couvercle (4) à l'aide d'une attache souple afin de servir de bouchon audit raccord (5) pour tubulure patient.

7. Ensemble selon la revendication 1, **caractérisé par** un raccord (9) d'échantillonnage/drainage adapté audit couvercle (4) pour manipuler le fluide aspiré.

8. Ensemble selon la revendication 1, **caractérisé en ce que** ledit raccord de vide (2) incorpore un conduit (10) via lequel on peut amener le vide à l'intérieur dudit récipient collecteur (1).

9. Structure munie d'un sac de prélèvement par aspiration pouvant être insérée dans un récipient collecteur (1) de fluides liquides qui est ouvert à l'une de ses extrémités et qui incorpore un raccord de vide (2) adapté pour communiquer avec une source de vide, la structure munie d'un sac de prélèvement par aspiration comprenant:
- un couvercle (4) pour fermer ladite une extrémité ouverte dudit récipient collecteur (1),
- un sac souple (3) de prélèvement par aspiration fixé audit couvercle (4),
- un raccord (5) pour tubulure patient prévu au niveau dudit couvercle et servant à fournir des communications fluidiques à partir de la source de fluides jusqu'à l'intérieur dudit sac (3) de prélèvement par aspiration,
- un conduit (7) prévu au niveau dudit couvercle (4) pour transmettre le vide appliqué depuis l'espace (11) restant entre la surface intérieure dudit récipient collecteur (1) et le couvercle (4) jusqu'à l'intérieur dudit sac (3) de prélèvement par aspiration, et
- un filtre (6) servant à prévenir toute pénétration de contaminants dans le système de vide,
**caractérisé en ce que**
- ledit filtre (6) est adapté à une surface du couvercle (4) tournée vers l'intérieur du sac (3) de prélèvement par aspiration,
- ledit filtre est adapté à l'extrémité dudit conduit débouchant dans l'intérieur dudit sac de prélèvement par aspiration,
- ledit couvercle (4) est une pièce unitaire.
